(19) Europäisches Patentamt
European Patent Office
Office européen des brevets

(11) **EP 4 166 086 A1**

(12) **EUROPEAN PATENT APPLICATION**
published in accordance with Art. 153(4) EPC

(43) Date of publication:
**19.04.2023 Bulletin 2023/16**

(51) International Patent Classification (IPC):
**A61B 6/08** (2006.01)   **G01S 1/00** (2006.01)

(21) Application number: **20941222.0**

(22) Date of filing: **02.12.2020**

(86) International application number:
**PCT/CN2020/133372**

(87) International publication number:
**WO 2021/253736 (23.12.2021 Gazette 2021/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.06.2020  CN 202010547154**

(71) Applicant: **CareRay Digital Medical Technology Co., Ltd.**
**Suzhou, Jiangsu 215123 (CN)**

(72) Inventors:
• **LIU, Yuhao**
  **Suzhou, Jiangsu 215123 (CN)**
• **LIU, Jianqiang**
  **Suzhou, Jiangsu 215123 (CN)**

(74) Representative: **Lavoix**
**Bayerstraße 83**
**80335 München (DE)**

(54) **REAL-TIME SPATIAL PRECISE MAGNETIC POSITIONING APPARATUS, RAY IMAGING SYSTEM, AND REAL-TIME SPATIAL PRECISE MAGNETIC POSITIONING METHOD**

(57)    A real-time spatial precise magnetic positioning apparatus, a ray imaging system, and a real-time spatial precise magnetic positioning method. The ray imaging system comprises a ray source, a beam limiting device, a flat panel detector, and a real-time spatial precise magnetic positioning apparatus. The magnetic positioning apparatus comprises a processor, a magnetic field generation apparatus and a magnetic sensor array, wherein the magnetic field generation apparatus and the beam limiting device are coaxially arranged; a plurality of magnetic sensors (4) of the magnetic sensor array are arranged on the flat panel detector in a distributed manner; the magnetic field generation apparatus at least comprises an alternating magnetic field generator (31) for generating an alternating magnetic field; the plurality of magnetic sensors (4) of the magnetic sensor array can independently measure a magnetic induction intensity, and can send data of the magnetic induction intensity, which is measured in real time, to the processor; and the processor determines the positional relationship between the beam limiting device and the flat panel detector according to the magnitude of the magnetic induction intensity measured by the magnetic sensors (4) in real time. Centimeter-level positioning precision in an indoor environment is realized; and the present invention is applicable to medical instruments and industrial control, and has a strong anti-interference capability.

FIG.1

## Description

### CROSS-REFERENCE TO RELATED APPLICATION

This application claims the benefit of priority to Chinese Patent Application No. 2020105471548, filed Jun. 16, 2020, which are hereby incorporated by reference herein in its entirety.

### TECHNICAL FIELD

The present disclosure generally relates to a medical equipment positioning field, and more particularly relates to a real-time spatial precise magnetic positioning device, a radiographic imaging system and a magnetic positioning method.

### BACKGROUND

The technologies currently used for positioning mainly comprise satellite positioning technology, wireless positioning technology (Wi-Fi positioning, Bluetooth positioning, etc.), environmental characteristic magnetic positioning technology, accelerometer positioning technology, etc.

The satellite positioning technology is to determine location of a positioning point by measuring the electromagnetic wave propagation time difference between the positioning point and different satellites, and then converting it into a distance from the positioning point to the satellite; the wireless positioning technology is to determine a position of a positioning point by measuring the wireless signal strength at the positioning point (the strength of Wi-Fi And Bluetooth and other signals); the environmental characteristic magnetic positioning technology is to determine a location of a positioning point by testing the environmental magnetic field where the positioning point is located, and comparing with a database; the accelerometer positioning technology is to obtain the trajectory of a positioning point by measuring acceleration of an object in real time, and integrating twice in time. At present, according to the field of application, these positioning technologies are combined to complement each other, forming various application schemes.

However, above positioning technologies have their own shortcomings: the satellite positioning technology tests relative position of the positioning point to each satellite, so it requires signals from more than three satellites to be received at the positioning point to determine the position, and it can only be applied outdoors and has a high cost; in addition, because speed of propagation of electromagnetic waves is very fast, even if atomic clock is used for time service, the positioning error is at meter level.

The wireless positioning technology and characteristic magnetic positioning technology can be applied indoors and outdoors, however, due to a need to compare test data of the positioning point with the database, it is necessary to carry out environmental mapping and database establishment before positioning, and can only be applied to known environment, and due to large interference, the positioning accuracy is not high, generally at meter level.

Since the accelerometer positioning technology can only determine relative movement of the test point, other technologies are needed to perform position calibration before starting positioning, such as starting movement at a specified position; in addition, because this technology only tests the acceleration over time, its error accumulates with increase of time, and generally, the positioning accuracy exceeds one meter after a few minutes.

However, the positioning accuracy of medical equipment needs to be at centimeter level, and there is no precise positioning technology that can be applied to medical equipment in an indoor environment in the conventional art.

### SUMMARY

In order to overcome the shortcomings of the prior art, the present disclosure provides a real-time spatial magnetic positioning device, a radiographic imaging system and a magnetic positioning method, which can be used indoors and have a positioning accuracy at centimeter level or even less than one centimeter, and technical solutions are as follows:

In one aspect, the present disclosure provides a real-time spatial magnetic positioning device for realizing the alignment of a first object to a reference area on a second object, the magnetic positioning device comprises a processor, a magnetic field generating device arranged coaxially with the first object, and a magnetic sensor array arranged on the second object, wherein the magnetic field generating device at least comprises an alternating magnetic field generator for generating an alternating magnetic field, and the magnetic sensor array is configured such that a distance from each magnetic sensor of the magnetic sensor array to a center point of the reference area is equal;

a plurality of magnetic sensors of the magnetic sensor array are capable of independently detecting magnetic induction intensity in real time, and sending real-time detected magnetic induction intensity data to the processor, and the processor is configured to compare values of the magnetic induction intensity detected in real time by respective magnetic sensors;

the position of the first object and/or the second object is configured to be adjusted until it is determined that the first object at a current position is aligned with the reference area on the second object after a comparison by the processor, if the magnetic induction intensity detected in real time by more than half of the magnetic sensors is equal, or differences of the magnetic induction intensity are less than a preset threshold or a proportional threshold.

As another technical solution, the present disclosure further provides a second real-time spatial magnetic positioning device for determining a position relationship between a first object and a reference area on a second object, the magnetic positioning device comprises a processor, a magnetic field generating device arranged coaxially with the first object, and a magnetic sensor array arranged on the second object, wherein the magnetic field generating device at least comprises an alternating magnetic field generator for generating an alternating magnetic field, and the magnetic sensor array is configured such that set positions of a plurality of magnetic sensors thereof have a certain position relationship with a center point of the reference area;

  a plurality of magnetic sensors of the magnetic sensor array are capable of independently detecting magnetic induction intensity in real time, and sending a real-time detected magnetic induction intensity data to the processor, and the processor is configured to calculate a position coordinate of respective magnetic sensors relative to the magnetic field generating device according to the magnetic induction intensity data;
  the processor is configured to calculate a position coordinate of the center point of the reference area relative to the first object based on the position coordinates of more than half of the magnetic sensors relative to the magnetic field generating device and position relationships between the magnetic sensors and the center point of the reference area.

Technical solution for the second real-time spatial magnetic positioning device, the position coordinate of the center point of the reference area relative to the first object calculated by the processor is a three-dimensional coordinates of x/y/z coordinate axes, and the processor is configured to analyze the three-dimensional coordinate: if the coordinates of two coordinate axes parallel to a plane where the first object is located are 0 or within a preset range close to 0, it is determined that the first object at the current position is aligned with the reference area on the second object.

Technical solutions for the above two real-time spatial magnetic positioning devices, further, the magnetic field generating device further comprises a bias magnet for biasing the magnetic sensors to a preset operating magnetic field range, and the bias magnet is a permanent magnet or an electromagnet.

Technical solutions for the above two real-time spatial magnetic positioning devices, further, the alternating magnetic field generator is capable of performing magnetic field encoding, and the magnetic field generating device is capable of bidirectional communication with the magnetic sensor array.

Further, the alternating magnetic field generator is configured to adjust the weakening of intensity of the magnetic field generated by itself according to the relative position relationship between the first object and the second object.

Optionally, the alternating magnetic field generator is configured to be any one of the following three manners:

  the alternating magnetic field generator comprises three orthogonal modulation coils, and by modulating the coil current, the magnetic field generated by the magnetic field generating device can change in the size and/or direction of the magnetic field in a three-dimensional space; or,
  the alternating magnetic field generator comprises a first modulation coil and a second modulation coil at a preset angle, and the two modulation coils work alternately; if the sensor is at a position where the magnetic field gradient of the first coil is less than 0.01 mT/m, the magnetic field gradient generated by the second coil at the position of the sensor must be greater than 0.01 mT/m due to the two coils have a preset angle, and at this moment, the magnetic field sensors obtain the relative position relationship between the first object and the second object depending on the magnetic field generated by the second coil; or,
  the alternating magnetic field generator comprises a permanent magnet and a mechanical transmission device for driving the permanent magnet to move, wherein the permanent magnet generates an alternating magnetic field in space under the drive of the mechanical transmission device, and the magnetic field can be modulated by the mechanical transmission device.

As a further first solution, the alternating magnetic field generator and the sensor array are respectively provided with an angle sensor, and the angle sensors are respectively used to calculate the azimuth angles of the first object and the second object while the magnetic sensors carry out magnetic field measurement, to determine the attitude between the first object and the second object.

As a further second solution, the alternating magnetic field generator and the sensor array are respectively provided with an acceleration sensor, and the acceleration sensors are respectively used to calculate three-dimensional acceleration data of the first object and the second object while the magnetic sensors carry out magnetic field measurement, to determine the acceleration, velocity and relative position relationship of the first object and the second object.

In another aspect, the present disclosure provides a radiographic imaging system with a magnetic positioning function, the system comprises a radiation source, a collimator, a flat panel detector, and a real-time spatial magnetic positioning device, wherein the magnetic positioning device comprises a processor, a magnetic field generating device and a magnetic sensor array, the magnetic field generating device is arranged coaxially with the collimator, a plurality of magnetic sensors of the magnetic sensor array is distributed on the flat panel detector;

the magnetic field generating device at least comprises an alternating magnetic field generator for generating an alternating magnetic field, the magnetic sensors of the magnetic sensor array is capable of independently detecting magnetic induction intensity in real time, and sending the real-time detected magnetic induction intensity data to the processor, and the processor determines the position relationship between the collimator and the flat panel detector according to the magnetic induction intensity detected by the respective magnetic sensors in real time.

In another aspect, the present disclosure provides a first real-time spatial magnetic positioning method, comprising following steps:

S11, arranging a magnetic field generating device coaxially with a first object, setting a reference area to be aligned on a second object, and determining a center point of the reference area; and arranging a plurality of magnetic sensors on the second object where a distance from each magnetic sensor to the center point is equal;

S12, generating an alternating magnetic field by the magnetic field generating device, and independently detecting magnetic induction intensity in real time by the magnetic sensors;

S 13, comparing the magnetic induction intensity detected in real time by the magnetic sensors;

S14, determining that the first object at a current position is aligned with the reference area on the second object if the magnetic induction intensity detected in real time by more than half of the magnetic sensors is equal, or differences of the magnetic induction intensity are less than a preset threshold or a proportional threshold, and ending positioning; otherwise, proceeding S15;

S15, adjusting position of the first object and/or the second object, and repeating Steps S12-S14.

In another aspect, the present disclosure provides a second real-time spatial magnetic positioning method, comprising following steps:

S21, arranging a magnetic field generating device coaxially with a first object, setting a reference area to be aligned on a second object, and determining a center point of the reference area; and arranging a plurality of magnetic sensors on the second object, and acquiring a position relationship between each magnetic sensor and the center point;

S22, generating an alternating magnetic field by the magnetic field generating device, and independently detecting magnetic induction intensity in real time by the magnetic sensors;

S23, calculating a position coordinate of the each magnetic sensor relative to the magnetic field generating device according to detection data of the magnetic induction intensity;

S24, excluding one or less than half of the magnetic sensors as an interfered magnetic sensor if the position coordinate of the one or less than half of the magnetic sensors deviate from a plane determined by the position coordinates of remaining magnetic sensors, and obtaining the position coordinate of the center point relative to the first object only according to the position coordinates of the remaining magnetic sensors relative to the magnetic field generating device and position relationships between the remaining magnetic sensors and the center point;

S25, determining that the first object at a current position is aligned with the reference area on the second object if in a three-dimensional coordinate of x/y/z coordinate axes of the center point relative to the first object, the coordinates of two coordinate axes parallel to a plane where the first object is located are 0 or within a preset range close to 0, and ending positioning; otherwise, proceeding S26;

S26, adjusting position of the first object and/or the second object, and repeating Steps S22-S25.

In another aspect, the present disclosure provides a third real-time spatial magnetic positioning method, comprising following steps:

S31, arranging two coils of a magnetic field generator at a preset angle on a first object, wherein the preset angle is not equal to 90°, setting a reference area to be aligned on a second object, and determining a center point of the reference area; and arranging a plurality of magnetic sensors on the second object, and acquiring a position relationship between each magnetic sensor and the center point;

S32, generating an alternating magnetic field by alternatively operating two coils of the magnetic field generator, and independently detecting magnetic induction intensity in real time by the sensors;

S33, counting one set when the two coils alternatively operate one time, dividing detection data of the magnetic induction intensity into several groups; respectively calculating a position coordinate of each magnetic sensors relative to the magnetic field generator according to the detection data of the magnetic induction intensity;

S34, if difference between two position coordinates in one group exceeds a preset threshold, discarding one position coordinate data with a larger offset by comparing with the data detected by other magnetic sensors;

S35, obtaining the position coordinates of the center point relative to the first object according to the remaining position coordinate data after processed in S34 and the position relationships between the remaining magnetic sensors and the center point;

S36, determining that the first object at the current position is aligned with the reference area on the second object if in a three-dimensional coordinate of x/y/z axes of the center point relative to the first object, the coordinates of two coordinate axes parallel to a plane where the first object is located are 0 or within a preset range close to 0, and ending positioning; otherwise, proceeding S37;

S37, adjusting the position of the first object and/or the second object, and repeating Steps S32-S36.

In addition, the present disclosure further provides a real-time spatial magnetic positioning correction method, comprising following steps:

S41, calculating a relative position of a first object relative to a second object at time t0 from a three-dimensional magnetic field data of magnetic sensors, according to the magnetic positioning method as described above;

S42, at time t1, obtaining three-dimensional magnetic field data, three-dimensional acceleration data, and three-dimensional attitude data at time t1, respectively through a magnetic sensor, an accelerometer, and an angle sensor;

S43, calculating a relative position pt1 of the first object relative to the second object at time t1 according to the three-dimensional magnetic field data and three-dimensional attitude data at time t1; calculating a relative speed v1 of the first object relative to the second object at time t1 according to the three-dimensional acceleration data at time t1;

S44, at time t2, obtaining three-dimensional magnetic field data, three-dimensional acceleration data, and three-dimensional attitude data at time t2, respectively through the magnetic sensor, the accelerometer, and the angle sensor;

S45, calculating the relative position pt2 of the first object relative to the second object at time t2 according to the three-dimensional magnetic field data and three-dimensional attitude data at time t2; calculating the relative position pt2' of the first object relative to the second object at time t2 according to the three-dimensional acceleration data at time t2 and the relative speed v1 at time t1;

S46, comparing pt2 and pt2', and determining the relative position of the first object relative to the second object at time t2 is the mean value of pt2 and pt2' if the error between the two is within 1 cm, otherwise proceeding S47-S48;

S47, comparing the three-dimensional acceleration data at time t2 with the speed v1 at time t1, determining the relative position of the first object relative to the second object at time t2 is pt2 if both the speed v1 and the three-dimensional acceleration are approximately 0, otherwise the relative position of the first object relative to the second object at time t2 is pt2';

S48, adjusting the position of the first object and/or the second object, and repeating Steps S41-S46.

The beneficial effects brought about by the technical solution provided in the present disclosure are as follows:

a. The positioning accuracy is less than lcm within a space of several meters;
b. Strong anti-interference ability is provided;
c. The cost is controllable, which is suitable for positioning and alignment of devices widely used in the fields of industrial control and medical equipment.

**BRIEF DESCRIPTION OF THE DRAWINGS**

In order to illustrate more clearly the technical solutions in the embodiments of the present disclosure, the following description of the accompanying drawings, which are necessary for the description of the embodiments, will be briefly described. It is obvious that the drawings in the following description are only some embodiments of the present disclosure. Other drawings may be obtained from these drawings without any creative work on the part of those skilled in the art.

FIG. 1 is a schematic diagram of the installation and application of a real-time spatial magnetic positioning device in a radiographic imaging system according to an embodiment of the present disclosure;
FIG. 2 is a schematic structure diagram of an alternating magnetic field generator comprising three orthogonal modulation coils in a magnetic positioning device provided by an embodiment of the present disclosure;
FIG. 3 is a schematic structure diagram of an alternating magnetic field generator comprising a mechanical transmission device and a permanent magnet in a magnetic positioning device provided by an embodiment of the present disclosure;
FIG. 4 is flowchart of a first magnetic positioning method provided by an embodiment of the present disclosure;
FIG. 5 is flowchart of a second magnetic positioning method provided by an embodiment of the present disclosure.

Wherein, the references are as follows: 1-first object, 2-second object, 31-alternating magnetic field generator, 32-bias magnet, 4-magnetic sensor.

## DETAILED DESCRIPTION

In order to enable those skilled in the art to better understand the solutions of the present disclosure, and to have a clearer understanding of the object, technical solutions and advantages of the present disclosure and its advantages, the technical solutions in the embodiments of the present disclosure are explained clearly and completely below in conjunction with specific examples and the accompanying drawings. It is noted that the implementations not illustrated or described in the accompanying drawings are in a form known to those skilled in the art. Further, while exemplary parameters containing specific values may be provided herein, it should be understood that the parameters need not be exactly equal to the corresponding values, but may approximate the corresponding values within acceptable error tolerances or design constraints. And apparently, the described embodiments are merely a part of the embodiments of the present disclosure, not all the embodiments. Based on the embodiments of the present disclosure, all other embodiments obtained by one of ordinary skill in the art without creative work fall within the protective scope of the present disclosure. In addition, the terms "comprise" and "have" and any variations thereof are intended to cover non-exclusive inclusions, for example, processes, methods, devices, products or equipment that include a series of steps or units are not necessarily limited to those clearly listed steps or units, but may include other steps or units not explicitly listed or inherent to these processes, methods, products or equipment.

The present disclosure puts forward a high-precision positioning scheme that uses a single magnet or a magnet array to cooperate with a magnetic sensor array. The magnet/magnet array comprises one or more permanent magnets or electromagnets or a combination thereof, the magnetic sensor array comprises a plurality of magnetic field sensors. In one embodiment, the magnet/magnet array can communicate wirelessly with the magnetic sensor array. This technology can provide a positioning accuracy of less than one centimeter within a range of several meters, which fills the gap of centimeter-level precision positioning, and has a wide range of applications in industrial control and medical equipment.

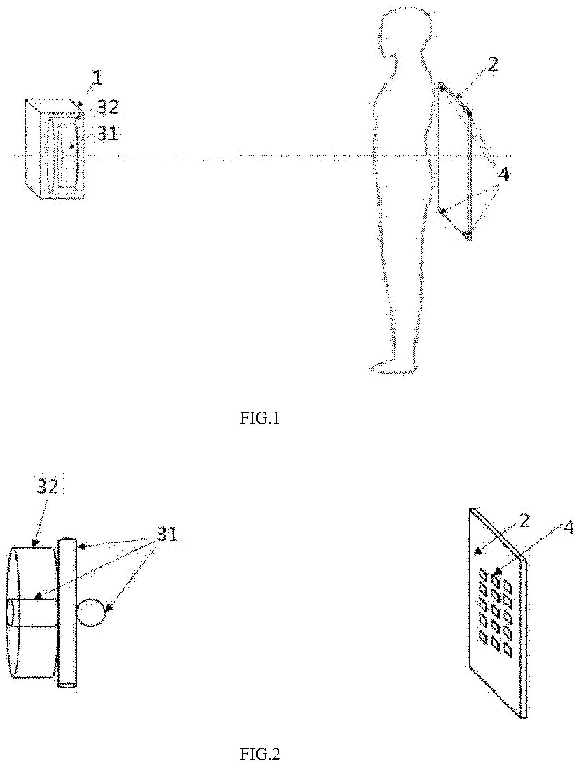

In one embodiment of the present disclosure, a real-time spatial magnetic positioning device is provided, which is used for realizing an alignment of a first object 1 to a reference area on a second object 2, as shown in FIG. 1, the magnetic positioning device comprises a processor, a magnetic field generating device arranged coaxially with the first object 1, and a magnetic sensor array arranged on the second object 2, the magnetic field generating device at least comprises an alternating magnetic field generator 31 for generating an alternating magnetic field, and the magnetic sensor array is configured such that a distance from each magnetic sensor 4 of the magnetic sensor array to a center point of the reference area is equal. Specifically, the alternating magnetic field generator 31 may have various forms:

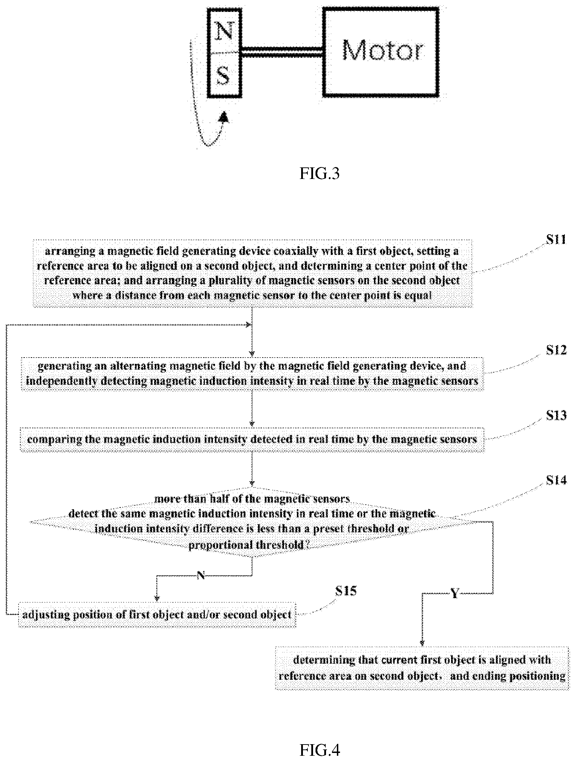

In one embodiment of the present disclosure, the alternating magnetic field generator 31 comprises a permanent magnet and a mechanical transmission device for driving the permanent magnet to move, and the magnetic field can be modulated by the mechanical transmission device, for example, the permanent magnet is fixed on a motor, as shown in FIG. 3, the permanent magnet can generate an alternating magnetic field in space when the motor drives the permanent magnet to rotate at a certain speed. By controlling the speed of the motor, the spatial magnetic field can be modulated. Further, the permanent magnet may be combined with a coil, so that advantages of the permanent magnet such as strong magnetic field and no power consumption are complementary and combined with the advantages of coil magnetic field such as convenient control, thereby reducing costs and improving positioning accuracy.

In another embodiment of the present disclosure, the alternating magnetic field generator 31 comprises three orthogonal modulation coils, as shown in FIG. 2, by modulating the coil current, the magnetic field generated by the magnetic field generating device can change in the size and/or direction of the magnetic field in a three-dimensional space. In this case, the following positioning methods can be used:

One possible positioning method is scanning. The magnetic field generated by the magnetic field generating device scans at a certain speed in three-dimensional space. When an axis of a magnetic sensor in the magnetic sensor array is exactly parallel to it, its value reaches a maximum. Since the magnetic sensor array and the magnetic field generating device can communicate with each other, the magnetic sensor array can calculate its position relative to the magnetic field generating device after scanning the magnetic field in entire space once.

Another possible positioning method is guiding. First, the magnetic sensors complete a search in entire space to find position of the magnetic sensors. Then the magnetic field generating device only scans in a small range to ensure that its direction (not necessarily the direction of the magnetic field, but only a conceptual direction, such as the opposite direction of the magnetic field, the vertical direction of the magnetic field, etc.) always points to the magnetic sensor array. Since there is no need to scan the magnetic field in entire space every time, this method can greatly improve the efficiency of positioning.

In addition to the alternating magnetic field generator, in an embodiment of the present disclosure, the magnetic field generating device further comprises a bias magnet 32 for biasing the magnetic sensors 4 to a preset operating magnetic field range, and the bias magnet 32 is a permanent magnet or an electromagnet. Specifically, the magnetic field generating device generates a non-uniform magnetic field in space, which comprises two parts: one part is a bias magnet field, this part of the magnetic field is generated by a permanent magnet or an electromagnet, and is used to bias the magnetic sensors to a suitable operating magnetic field range; the other part is an alternating magnetic field, which is generated by an alternating current coil or a moving magnet, frequency of the alternating magnetic field can be controlled by controlling the alternating current or the movement frequency of the mechanical structure that controls the movement of the magnet to perform magnetic field encoding. It should be noted that the bias magnetic field can be integrated in the alternating magnetic field generator, that is, a constant current is superimposed on the alternating current; if the magnetic sensor does not require a bias magnetic field to perform high-precision measurement, the bias magnet 32 can also be omitted.

A plurality of magnetic sensors 4 of the magnetic sensor array can independently detect magnetic induction intensity in real time, and send the real-time detected magnetic induction intensity data to the processor, and the processor compares the magnetic induction intensity data detected by the respective magnetic sensors 4 in real time;

The position of the first object 1 and/or the second object 2 is adjusted until: after comparison by the processor, result is obtained that magnetic induction intensity data detected in real time by more than half of the magnetic sensors is the same or the difference between detected magnetic induction intensity is less than a preset threshold or a proportional threshold, specifically for example, if magnetic induction intensity difference between a maximum magnetic induction intensity and a minimum magnetic induction intensity is less than 1 mGs, or the ratio of magnetic induction intensity difference to a current average magnetic induction intensity is less than 1%, it is determined that the first object 1 at a current position is aligned with the reference area on the second object 2.

Specifically, the second object 2 preferably has a flat surface, and the reference area to be aligned with the first object 1 is provided on the flat surface, and the center point of the reference area is determined. Taking the application of the magnetic positioning device in a radiographic imaging system as an example, the radiographic imaging system comprises a radiation source, a collimator, and a flat panel detector, wherein the magnetic positioning device comprises a processor, a magnetic field generating device and a magnetic sensor array, the magnetic field generating device of the magnetic positioning device is arranged coaxially with the collimator (namely, the collimator is the first object 1), one bias coil and one alternating magnetic field coil can be selected and arranged coaxially, in this configuration, the magnet will generate a centrosymmetric spatial magnetic field with the X-ray beam as the axis in space; a plurality of magnetic sensors 4 of the magnetic sensor array are distributed on the flat panel detector (namely, the flat panel detector is the second object 2), and it is not limited to the number and position of the magnetic sensors 4 as four magnetic sensors 4 are located at the four corners of a flat panel as shown in FIG. 1.

As shown in FIG. 1, taking the arrangement of the four magnetic sensors as an example, when the positioning starts, the four magnetic sensors work at the same time, and the amplitude of the magnetic field generated by the magnetic field generator is demodulated through a phase-locked filtering and amplification technology. According to the amplitude, the positions of the respective magnetic sensors relative to the collimator can be calculated. Due to the use of 4 magnetic sensors, if one of them is interfered so as that the magnetic field amplitude test is inaccurate, the position of the flat panel can also be determined through the remaining three (magnetic sensors). In order to further improve the anti-interference ability of the system, an array comprising more magnetic sensors can be provided, for example, eight magnetic sensors can be provided to form an array, and the position could still be positioned accurately if (at most) three sensors operate in interference, relevant details are as follows.

The flat panel detector needs to be close to the area to be photographed when shooting, and center line of the X-ray beam is required to be aligned and perpendicular to the center area of the flat panel detector (the area near the center of the flat panel). This requires physician to accurately determine the placing position of the flat panel detector relative to the X-ray source (collimator), and the magnetic positioning technical solution provided by the embodiment of the present disclosure can provide positioning guidance to the physician, main inventive concept is as follows: the magnetic field generating device at least comprises an alternating magnetic field generator for generating an alternating magnetic field, a plurality of magnetic sensors of the magnetic sensor array can independently detect magnetic induction intensity in real time, and send the real-time detected magnetic induction intensity data to the processor, and the processor is configured to determine the position relationship between the collimator and the flat panel detector according to the magnetic induction intensity data detected by the respective magnetic sensors in real time.

In order to further improve the reliability of positioning, the alternating magnetic field generator 31 can perform magnetic field encoding, the magnetic field generating device and the magnetic sensor array can communicate bidirectionally, and the alternating magnetic field generator 31 can adjust the magnetic field intensity generated by itself according to the determined relative position of the first object and the second object for several times; the method for determining the relative position of the two objects is specifically described as follows, for example, adjusting the magnetic field generated by the alternating magnetic field generator 31 to become weaker. If the magnetic sensor array senses that the magnetic field of each axis of each magnetic sensor is weak, the magnetic sensor array can notify the magnetic field generating device through wireless communication, that is, the magnetic field generating device is notified to increase the magnetic field intensity; on the contrary, if the magnetic field magnetic sensor array senses that the magnetic field is too strong and exceeds its measuring range, the magnetic field generating device is notified to reduce the magnetic field.

In an embodiment of the present disclosure, a real-time spatial magnetic positioning method is provided, as shown in FIG. 4, the magnetic positioning method comprises following steps:

S11, arranging a magnetic field generating device coaxially with a first object, setting a reference area to be aligned on a second object, and determining a center point of the reference area; and arranging a plurality of magnetic sensors on the second object where a distance from each magnetic sensor to the center point are the same;

S12, generating an alternating magnetic field by the magnetic field generating device, and independently detecting the magnetic induction intensity in real time by the magnetic sensors;

S13, comparing the magnetic induction intensity data detected by the magnetic sensors in real time;

S14, determining that the first object at a current position is aligned with the reference area on the second object if the magnetic induction intensity data detected in real time by more than half of the magnetic sensors is equal, or differences of the magnetic induction intensity data are less than a preset threshold or a proportional threshold, then ending positioning; otherwise, proceeding S15;

S15, adjusting position of the first object and/or the second object, and repeating Steps S12-S14.

It can be seen from the above that when all the magnetic sensors are arranged at the same distance from the center point of the reference area to be aligned with the first object on the second object, it can be known from the geometric relationship that one plane can be determined by three points, therefore, the number of the magnetic sensors is greater than or equal to three. In the case of more than or equal to three, only when the magnetic field generating device is directly facing the center of the circle where the three or more magnetic sensors are located, it is possible that the magnetic induction intensity detected by the magnetic sensors in real time are the same. In actual operation, the most ideal situation is that the first object is completely facing the center point of the second object; in a case that a certain deviation is allowed, when difference of the magnetic induction intensity detected by the magnetic sensor in real time is less than a preset threshold or proportional threshold, it should also be used as the basis for determining the alignment, for example, the maximum magnetic induction intensity among the data detected by all the magnetic sensors subtracts the minimum magnetic induction intensity to obtain the magnetic induction intensity difference, and if this magnetic induction intensity difference is less than 1 mGs, or the ratio of the magnetic induction intensity difference to a current average magnetic induction intensity is less than 1%, it is determined that the first object 1 at the current position (within the allowable error range) is aligned with the reference area on the second object 2.

Preferably, the number of magnetic sensors is provided to be three or more, such as four, so that even one of four magnetic sensors is interfered, the magnetic positioning will not be affected; or eight magnetic sensors are provided, if three of them are interfered, it is still possible to determine whether the first object is aligned with the second object based on the remaining five magnetic sensors that are not interfered. Specifically, for example, if six of the eight magnetic sensors have the same magnitude of magnetic induction intensity detected in real time, but the other two have different values of magnetic induction intensity, it can be determined that the two magnetic sensors are interfered, and the interfered detection results thereof can be ruled out.

The technical solution of the above embodiment limits the distance between each magnetic sensor and the center point to be the same, and under this condition, the distance and coordinate values between the specific magnetic sensors (or center point) and the magnetic field generating device may not be considered. In the following embodiment of the present disclosure, a real-time spatial magnetic positioning device based on another inventive concept is provided for determining a position relationship between a first object 1 and a reference area on a second object 2, the magnetic positioning device comprises a processor, a magnetic field generating device arranged coaxially with the first object 1, and a magnetic sensor array arranged on the second object 2, the magnetic field generating device at least comprises an alternating magnetic field generator 31 for generating an alternating magnetic field, and the magnetic sensor array is configured such that the set positions of a plurality of magnetic sensors 4 thereof have a certain position relationship with a center point of the reference area;

In this embodiment, the positions of the magnetic sensors 4 have be arranged discretionarily, and then the position relationship between the magnetic sensors 4 and the center point could be determined. For example, in a pre-established coordinate system, taking a certain magnetic sensor 4 as an origin, the coordinate of the center point is $(x1, y1, z1)$.

A plurality magnetic sensors 4 of the magnetic sensor array can independently detect magnetic induction intensity in real time, and send the real-time detected magnetic induction intensity data to the processor, and since the frequency of the magnetic field change is known, technologies such as phase-locked amplification can be used to filter and suppress noise to obtain high-precision magnetic field measurement values, and the processor may obtain the positions of the magnetic sensors relative to the magnetic field generating device, that is, the position coordinates of the respective magnetic sensors 4 relative to the magnetic field generating device through corresponding calculations according to the detection results of the respective magnetic sensors. The position coordinates here are based on the same coordinate system as the above (the coordinate system with the same x/y/z axis direction is called the same coordinate system), with the magnetic field generating device as an origin, and the coordinate of the certain magnetic sensor is (x2, y2, z2), then the coordinate of the center point relative to the magnetic field generator is (x1+x2, y1+y2, z1+z2).

Specifically, the magnetic field amplitude can be obtained by real-time detection of the magnetic induction intensity by the magnetic sensor, one of classic algorithms for calculating the position of each magnetic sensor relative to the magnetic field generating device based on the amplitude is the Biot-Savart Law, that is the magnitude of the magnetic induction intensity dB generated by a current element Idl at a certain point P in space is directly proportional to the size of the current element Idl, is directly proportional to the sine of the angle between the position vector from the location of the current element Idl to point P and the current element Idl, and is inversely proportional to the square of the distance from the current element Idl to point P. The classic formula is as follows:

$$d\vec{B} = \frac{\mu_0}{4\pi} \frac{Idl \times \vec{r}}{r^3} = \frac{\mu_0}{4\pi} \frac{Idl\sin\theta}{r^2}$$

$$\vec{B} = \int_L \frac{\mu_0 I}{4\pi} \frac{dl \times \vec{e_r}}{r^2}$$

where, I is a source current, L is an integration path, dl is a micro line element of the source current, $\vec{e_r}$ is a unit vector of a current element pointing to the field point to be sought, $\mu_0$ is vacuum permeability, and its value is $4\pi \times 10^{-7} N/A^2$, direction of dB is perpendicular to a plane determined by Idl and $\vec{e_r}$, r is distance between the magnetic sensor and the magnetic field generating device, and $\vec{r}$ is a vector directed from the magnetic sensor to the magnetic field generating device.

The vector $\vec{r}$ calculated by the above formula can be converted into a coordinate in the coordinate system.

If the coil is far enough from the magnetic sensor, the following approximation can be used:

$$\frac{\mu_0}{4\pi} \left( \frac{3(\mathbf{m} \cdot \mathbf{r})\mathbf{r}}{r^5} - \frac{\mathbf{m}}{r^3} \right),$$

where, m=NSIn,

where, N is number of turns of the coil, S is area of the coil, I is the current of the coil, and n is direction, which is along the axis of the coil and perpendicular to the coil surface.

In principle, only one magnetic sensor is needed to determine the positional relationship of the center point relative to the magnetic field generating device. However, in order to improve the positioning accuracy and prevent the magnetic sensor from being interfered and causing the wrong positioning result, the magnetic sensor array is preferably provided with three or more magnetic sensors, and if one magnetic sensor is interfered, it will not affect the position determination of the whole array, with high stability.

The processor obtains the position coordinate of the center point of the reference area relative to the first object 1 based on the position coordinates of more than half of the magnetic sensors 4 relative to the magnetic field generating device and the position relationships between the magnetic sensors 4 and the center point of the reference area. For example, the number of the magnetic sensors is six, the coordinates of the center point obtained according to the real-time detection results of four magnetic sensors are all (x', y', z'), but the coordinates of the center point obtained according to the real-time detection results of the other two magnetic sensors are different, it can be determined that the two magnetic sensors are interfered, and the interfered detection results thereof can be ruled out.

For this technical solution of the real-time spatial magnetic positioning device, the processor obtains the position coordinate of the center point of the reference area relative to the first object 1 as three-dimensional coordinate of x/y/z coordinate axes, and the processor analyzes the three-dimensional coordinate, if the coordinates of two coordinate axes parallel to a plane where the first object 1 is located are 0, it is determined that the first object 1 at the current position is aligned with the reference area on the second object 2. In actual operation, it is the most ideal situation that the first object is completely facing the center point of the second object, for example, the coordinate system is established with the magnetic field generating device as an origin, and the center line of the collimator as the x-axis (or as the y-axis or z-axis), then when the coordinate value of the center point is (50, 0, 0), it means that the first object 1 is currently facing the center point of the second object 2; when a certain deviation is allowed, for example, in the coordinate values of the center point, the coordinate values of the y-axis and the z-axis are within a preset range close to 0, such as the range of [-5, +5], for example, the obtained coordinates of the center point corresponding to different magnetic sensors are (50, 5, -5), (50, -5, 5), (50, 5, 5), (50, -5, -5) and so on, it can be determined the first object 1 at the current position (within the allowed error range) is aligned with the reference area on the second object 2.

Same as the previous embodiment, in addition to the alternating magnetic field generator, the magnetic field generating device of this embodiment further comprises a bias magnet 32 for biasing the magnetic sensors 4 to a preset operating magnetic field range, and the bias magnet 32 is a permanent magnet or an electromagnet.

In order to further improve the reliability of positioning, the alternating magnetic field generator 31 can perform magnetic field encoding; the magnetic field generating device and the magnetic sensor array can communicate bidirectionally, and the alternating magnetic field generator 31 can adjust the magnetic field intensity generated by the alternating magnetic field generator according to the determined relative position of the first object and the second object for several times, for example, adjusting the magnetic field generated by the alternating magnetic field generator 31 to become weaker. When the magnetic sensor array senses that the magnetic field of each axis of each magnetic sensor is weak, the magnetic sensor array can notify the magnetic field generating device through wireless communication, that is the magnetic field generating device is notified to increase the magnetic field intensity; on the contrary, when the magnetic field magnetic sensor array senses that the magnetic field is too strong and exceeds its measuring range, the magnetic field generating device is notified to reduce the magnetic field.

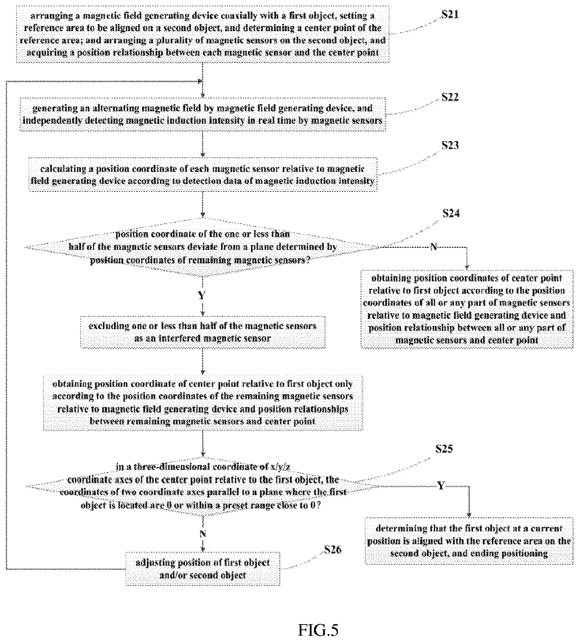

In an embodiment of the present disclosure, another real-time spatial magnetic positioning method is provided, as shown in FIG. 5, it comprises following steps:

> S21, arranging a magnetic field generating device coaxially with a first object, setting a reference area to be aligned on a second object, and determining a center point of the reference area; and arranging a plurality of magnetic sensors on the second object, and acquiring the position relationship between the respective magnetic sensors and the center point;
> S22, generating an alternating magnetic field by the magnetic field generating device, and independently detecting the magnetic induction intensity in real time by the magnetic sensors;
> S23, calculating the position coordinates of the respective magnetic sensors relative to the magnetic field generating device according to the detection data of the magnetic induction intensity;
> S24, excluding one or less than half of the magnetic sensors as the interfered magnetic sensors if the position coordinates of the one or less than half of the magnetic sensors deviate from the plane determined by the position coordinates of the remaining magnetic sensors, and obtaining the position coordinates of the center point relative to the first object only according to the position coordinates of the remaining magnetic sensors relative to the magnetic field generating device and the position relationships between the remaining magnetic sensors and the center point; if there is no deviation, that is, all the magnetic sensors are on the same plane, obtaining the position coordinates of the center point relative to the first object according to the position coordinates of all or any part of the magnetic sensors relative to the magnetic field generating device and the position relationship between all or any part of the magnetic sensors and the center point.

> S25, determining that the first object at the current position is aligned with the reference area on the second object if in a three-dimensional coordinate of x/y/z coordinate axes of the center point relative to the first object, the coordinates of two coordinate axes parallel to a plane where the first object is located are 0 or within a preset range close to 0, and ending positioning, specifically see the above for details; otherwise, proceeding S26;
> S26, adjusting the position of the first object and/or the second object, and repeating Steps S22-S25.

It should be noted that the above-mentioned magnetic positioning method embodiment belongs to the same concept as the magnetic positioning device provided in the above-mentioned embodiment, for the specific implementation process, please refer to the device embodiment, that is, all the features in the above-mentioned device embodiment can be introduced into the method embodiments by reference.

The embodiment of the present disclosure proposes a third structural form for the alternating magnetic field generator 31, that is the alternating magnetic field generator 31 comprises two modulation coils at a preset angle, the two modulation coils work alternately, and if the magnetic field is located at an area where the gradient of one coil is less than 0.01 mT/m, the relative position between the first object 1 and the second object 2 can be obtained according to another coil.

Specifically, the alternating magnetic field generator 31 and the sensor array each have an angle sensor, and the angle sensors are respectively used to calculate the azimuth angles of the first object 1 and the second object 2 while the magnetic sensors are measuring the magnetic field, to determine the attitude between two objects; and/or

the alternating magnetic field generator 31 and the sensor array each have an acceleration sensor, and the acceleration sensors are respectively used to calculate three-dimensional acceleration data of the first object 1 and the second object 2 while the magnetic sensors are measuring the magnetic field, to determine the acceleration, velocity and relative position of the first object 1 and the second object 2, for correcting the magnetic positioning, and the specific correction method is described in detail below.

The above method for obtaining the relative position between the first object 1 and the second object 2 according to another coil specifically comprises following steps:

S31, arranging two coils of a magnetic field generator at a preset angle on a first object, the preset angle is not equal to 90°, setting a reference area to be aligned on a second object, and determining a center point of the reference area; and arranging a plurality of magnetic sensors on the second object, and acquiring the position relationship between the respective magnetic sensors and the center point;

S32, generating an alternating magnetic field by alternatively operating two coils of a magnetic field generator, and independently detecting the magnetic induction intensity in real time by the magnetic sensors;

S33, counting one set when the two coils alternatively operate one time, dividing the detection data of the magnetic induction intensity into several groups; respectively calculating the position coordinates of the respective magnetic sensors relative to the magnetic field generator according to the detection data of the magnetic induction intensity;

S34, comparing with the data detected by other magnetic sensors, if the difference between the two position coordinates in a group exceeds a preset threshold, discarding position coordinate data with a larger offset;

S35, obtaining the position coordinates of the center point relative to the first object according to the remaining position coordinate data after processed in S34 and the position relationships between the remaining magnetic sensors and the center point;

S36, determining that the first object at the current position is aligned with the reference area on the second object if in a three-dimensional coordinate of x/y/z axes of the center point relative to the first object, the coordinates of two coordinate axes parallel to a plane where the first object is located are 0 or within a preset range close to 0, and ending positioning; otherwise, proceeding S37;

S37, adjusting the position of the first object and/or the second object, and repeating Steps S32-S36.

The method for correcting magnetic positioning described above comprises following steps:

S41, using the magnetic positioning method as described above, using the three-dimensional magnetic field data of the magnetic sensors to calculate the relative position of the first object relative to the second object at time t0;

S42, at time t1, using a magnetic sensor, an accelerometer, and an angle sensor to obtain three-dimensional magnetic field data, three-dimensional acceleration data, and three-dimensional attitude data at time t1, respectively;

S43, calculating a relative position pt1 of the first object relative to the second object at time t1 according to the three-dimensional magnetic field data and three-dimensional attitude data at time t1; calculating a relative speed v1 of the first object relative to the second object at time t1 according to the three-dimensional acceleration data at time t1;

S44, at time t2, using the magnetic sensor, the accelerometer, and the angle sensor to obtain three-dimensional magnetic field data, three-dimensional acceleration data, and three-dimensional attitude data at time t2, respectively;

S45, calculating the relative position pt2 of the first object relative to the second object at time t2 according to the three-dimensional magnetic field data and three-dimensional attitude data at time t2; according to the three-dimensional acceleration data at time t2 and the relative speed v1 at time t1, calculating the relative position pt2' of the first object relative to the second object at time t2;

S46, comparing pt2 and pt2', and determining the relative position of the first object relative to the second object at time t2 is the mean value of pt2 and pt2' if the error between the two is within 1 cm, otherwise proceeding S47-S48;

S47, comparing the three-dimensional acceleration value at time t2 with the velocity v1 at time t1, determining the relative position of the first object relative to the second object at time t2 is pt2 if both the velocity v1 and the three-dimensional acceleration are approximately 0, otherwise it is pt2';

S48, adjusting the position of the first object and/or the second object, and repeating Steps S41-S46.

The present disclosure can allow a plurality of magnetic sensor arrays to be positioned at the same time, that is, to realize the positioning of a plurality of objects. This is because different magnetic sensor arrays can independently communicate with the magnetic field generating device to determine their positions relative to the magnetic field generating device. In order to better control the spatial magnetic field and improve the positioning accuracy, a plurality of magnetic field generators can also be used and placed in different spatial positions.

Some magnetically sensitive devices, such as cardiac pacemakers, cannot be used in magnetic positioning, this is because although a weak magnetic field is used, the magnetic field may still be larger than the geomagnetic field, especially near the magnetic field generating device. For this reason, additional magnetic sensor arrays can be mounted near these magnetic field-sensitive devices. As mentioned above, this technology can use one magnetic field generating device to locate a plurality of magnetic sensor arrays, therefore, the position of magnetic sensitive devices can be determined in real time during positioning, and by actively reducing the magnetic field near the magnetic sensitive device to the level of the geomagnetic field, to guarantee it is not affected.

The foregoing is only a preferred embodiment of the present disclosure and is not intended to limit the present disclosure. Any modifications, equivalent substitutions, improvements, etc. made within the spirit and principles of the present disclosure shall be included within the scope of protection of the present disclosure.

**Claims**

1. A real-time spatial magnetic positioning device, wherein the magnetic positioning device is used for realizing the alignment of a first object (1) to a reference area on a second object (2), the magnetic positioning device comprises a processor, a magnetic field generating device arranged coaxially with the first object (1), and a magnetic sensor array arranged on the second object (2), the magnetic field generating device at least comprises an alternating magnetic field generator (31) for generating an alternating magnetic field, and the magnetic sensor array is configured such that a distance from each magnetic sensor (4) of the magnetic sensor array to a center point of the reference area is equal;

   a plurality of magnetic sensors (4) of the magnetic sensor array are capable of independently detecting magnetic induction intensity in real time, and sending real-time detected magnetic induction intensity data to the processor, and the processor is configured to compare values of the magnetic induction intensity detected in real time by respective magnetic sensors (4); the position of the first object (1) and/or the second object (2) is configured to be adjusted until it is determined that the first object (1) at a current position is aligned with the reference area on the second object (2) after a comparison by the processor, if the magnetic induction intensity detected in real time by more than half of the magnetic sensors (4) is equal, or differences of the magnetic induction intensity are less than a preset threshold or a proportional threshold.

2. A real-time spatial magnetic positioning device, wherein the magnetic positioning device is used for determining a position relationship between a first object (1) and a reference area on a second object (2), the magnetic positioning device comprises a processor, a magnetic field generating device arranged coaxially with the first object (1), and a magnetic sensor array arranged on the second object (2), wherein the magnetic field generating device at least comprises an alternating magnetic field generator (31) for generating an alternating magnetic field, and the magnetic sensor array is configured such that set positions of a plurality of magnetic sensors (4) thereof have a certain position relationship with a center point of the reference area;

   a plurality of magnetic sensors (4) of the magnetic sensor array are capable of independently detecting magnetic induction intensity in real time, and sending a real-time detected magnetic induction intensity data to the processor, and the processor is configured to calculate a position coordinate of respective magnetic sensors (4) relative to the magnetic field generating device according to the magnetic induction intensity data;

   the processor is configured to calculate a position coordinate of the center point of the reference area relative to the first object (1) based on the position coordinates of more than half of the magnetic sensors (4) relative to the magnetic field generating device and position relationships between the magnetic sensors (4) and the center point of the reference area.

3. The real-time spatial magnetic positioning device according to claim 2, wherein the position coordinate of the center point of the reference area relative to the first object (1) calculated by the processor is a three-dimensional coordinates of x/y/z coordinate axes, and the processor is configured to analyze the three-dimensional coordinate: if the coordinates of two coordinate axes parallel to a plane where the first object (1) is located are 0 or within a preset range close to 0, it is determined that the first object (1) at the current position is aligned with the reference area on the second object (2).

4. The real-time spatial magnetic positioning device according to claim 1 or 2, wherein the magnetic field generating device further comprises a bias magnet (32) for biasing the magnetic sensors (4) to a preset operating magnetic field range, and the bias magnet (32) is a permanent magnet or an electromagnet.

5. The real-time spatial magnetic positioning device according to claim 1 or 2, wherein the alternating magnetic field generator (31) is capable of performing magnetic field encoding, and the magnetic field generating device is capable of bidirectional communication with the magnetic sensor array.

6. The real-time spatial magnetic positioning device according to claim 1 or 2, wherein the alternating magnetic field generator (31) is configured to adjust the weakening of intensity of the magnetic field generated by itself according to the relative position relationship between the first object (1) and the second object (2).

7. The real-time spatial magnetic positioning device according to claim 1 or 2, wherein the alternating magnetic field generator (31) is configured to be any one of the following three manners:

the alternating magnetic field generator (31) comprises three orthogonal modulation coils, and by modulating the coil current, the magnetic field generated by the magnetic field generating device can change in the size and/or direction of the magnetic field in a three-dimensional space; or,

the alternating magnetic field generator (31) comprises a first modulation coil and a second modulation coil at a preset angle, and the two modulation coils work alternately; if the sensor (4) is at a position where the magnetic field gradient of the first coil is less than 0.01 mT/m, the magnetic field gradient generated by the second coil at the position of the sensor must be greater than 0.01 mT/m due to the two coils have a preset angle, and at this moment, the magnetic field sensors obtain the relative position relationship between the first object (1) and the second object (2) depending on the magnetic field generated by the second coil; or,

the alternating magnetic field generator (31) comprises a permanent magnet and a mechanical transmission device for driving the permanent magnet to move, wherein the permanent magnet generates an alternating magnetic field in space under the drive of the mechanical transmission device, and the magnetic field can be modulated by the mechanical transmission device.

8.   The real-time spatial magnetic positioning device according to claim 1 or 2, wherein the alternating magnetic field generator (31) and the sensor array are respectively provided with an angle sensor, and the angle sensors are respectively used to calculate the azimuth angles of the first object (1) and the second object (2) while the magnetic sensors carry out magnetic field measurement, to determine the attitude between the first object and the second object.

9.   The real-time spatial magnetic positioning device according to claim 1 or 2, wherein the alternating magnetic field generator (31) and the sensor array are respectively provided with an acceleration sensor, and the acceleration sensors are respectively used to calculate three-dimensional acceleration data of the first object (1) and the second object (2) while the magnetic sensors carry out magnetic field measurement, to determine the acceleration, velocity and relative position relationship of the first object (1) and the second object (2).

10.   A radiographic imaging system with a magnetic positioning function, wherein the system comprises a radiation source, a collimator, a flat panel detector, and a real-time spatial magnetic positioning device, wherein the magnetic positioning device comprises a processor, a magnetic field generating device and a magnetic sensor array, the magnetic field generating device is arranged coaxially with the collimator, a plurality of magnetic sensors (4) of the magnetic sensor array is distributed on the flat panel detector; The magnetic field generating device at least comprises an alternating magnetic field generator (31) for generating an alternating magnetic field, the magnetic sensors (4) of the magnetic sensor array is capable of independently detecting magnetic induction intensity in real time, and sending the real-time detected magnetic induction intensity data to the processor, and the processor determines the position relationship between the collimator and the flat panel detector according to the magnetic induction intensity detected by the respective magnetic sensors (4) in real time.

11.   A first real-time spatial magnetic positioning method, comprising following steps:

S11, arranging a magnetic field generating device coaxially with a first object, setting a reference area to be aligned on a second object, and determining a center point of the reference area; and arranging a plurality of magnetic sensors on the second object where a distance from each magnetic sensor to the center point is equal;

S12, generating an alternating magnetic field by the magnetic field generating device, and independently detecting magnetic induction intensity in real time by the magnetic sensors;

S13, comparing the magnetic induction intensity detected in real time by the magnetic sensors;

S14, determining that the first object at a current position is aligned with the reference area on the second object if the magnetic induction intensity detected in real time by more than half of the magnetic sensors is equal, or differences of the magnetic induction intensity are less than a preset threshold or a proportional threshold, and ending positioning; otherwise, proceeding 515;

S15, adjusting position of the first object and/or the second object, and repeating Steps S12-S14.

12.   A real-time spatial magnetic positioning method, comprising following steps:

S21, arranging a magnetic field generating device coaxially with a first object, setting a reference area to be aligned on a second object, and determining a center point of the reference area; and arranging a plurality of magnetic sensors on the second object, and acquiring a position relationship between each magnetic sensor and the center point;

S22, generating an alternating magnetic field by the magnetic field generating device, and independently detecting magnetic induction intensity in real time by the magnetic sensors;

S23, calculating a position coordinate of the each magnetic sensor relative to the magnetic field generating device according to detection data of the magnetic induction intensity;

S24, excluding one or less than half of the magnetic sensors as an interfered magnetic sensor if the position coordinate of the one or less than half of the magnetic sensors deviate from a plane determined by the position coordinates of remaining magnetic sensors, and obtaining the position coordinate of the center point relative to the first object only according to the position coordinates of the remaining magnetic sensors relative to the magnetic field generating device and position relationships between the remaining magnetic sensors and the center point;

S25, determining that the first object at a current position is aligned with the reference area on the second object if in a three-dimensional coordinate of x/y/z coordinate axes of the center point relative to the first object, the coordinates of two coordinate axes parallel to a plane where the first object is located are 0 or within a preset range close to 0, and ending positioning; otherwise, proceeding S26;

S26, adjusting position of the first object and/or the second object, and repeating Steps S22-S25.

13. A real-time spatial magnetic positioning method, comprising following steps:

S31, arranging two coils of a magnetic field generator at a preset angle on a first object, wherein the preset angle is not equal to 90°, setting a reference area to be aligned on a second object, and determining a center point of the reference area; and arranging a plurality of magnetic sensors on the second object, and acquiring a position relationship between each magnetic sensor and the center point;

S32, generating an alternating magnetic field by alternatively operating two coils of the magnetic field generator, and independently detecting magnetic induction intensity in real time by the sensors;

S33, counting one set when the two coils alternatively operate one time, dividing detection data of the magnetic induction intensity into several groups; respectively calculating a position coordinate of each magnetic sensors relative to the magnetic field generator according to the detection data of the magnetic induction intensity;

S34, if difference between two position coordinates in one group exceeds a preset threshold, discarding one position coordinate data with a larger offset by comparing with the data detected by other magnetic sensors;

S35, obtaining the position coordinates of the center point relative to the first object according to the remaining position coordinate data after processed in S34 and the position relationships between the remaining magnetic sensors and the center point;

S36, determining that the first object at the current position is aligned with the reference area on the second object if in a three-dimensional coordinate of x/y/z axes of the center point relative to the first object, the coordinates of two coordinate axes parallel to a plane where the first object is located are 0 or within a preset range close to 0, and ending positioning; otherwise, proceeding S37;

S37, adjusting the position of the first object and/or the second object, and repeating Steps S32-S36.

14. A real-time spatial magnetic positioning correction method, comprising following steps:

S41, calculating a relative position of a first object relative to a second object at time t0 from a three-dimensional magnetic field data of magnetic sensors, according to the magnetic positioning method according to any one of claims 11 to 13;

S42, at time t1, obtaining three-dimensional magnetic field data, three-dimensional acceleration data, and three-dimensional attitude data at time t1, respectively through a magnetic sensor, an accelerometer, and an angle sensor;

S43, calculating a relative position pt1 of the first object relative to the second object at time t1 according to the three-dimensional magnetic field data and three-dimensional attitude data at time t1; calculating a relative speed v1 of the first object relative to the second object at time t1 according to the three-dimensional acceleration data at time t1;

S44, at time t2, obtaining three-dimensional magnetic field data, three-dimensional acceleration data, and three-dimensional attitude data at time t2, respectively through the magnetic sensor, the accelerometer, and the angle sensor;

S45, calculating the relative position pt2 of the first object relative to the second object at time t2 according to the three-dimensional magnetic field data and three-dimensional attitude data at time t2; calculating the relative position pt2' of the first object relative to the second object at time t2 according to the three-dimensional acceleration data at time t2 and the relative speed v1 at time t1;

S46, comparing pt2 and pt2', and determining the relative position of the first object relative to the second object at time t2 is the mean value of pt2 and pt2' if the error between the two is within 1 cm, otherwise proceeding S47-S48;

S47, comparing the three-dimensional acceleration data at time t2 with the speed v1 at time t1, determining the relative position of the first object relative to the second object at time t2 is pt2 if both the speed v1 and the three-dimensional acceleration are approximately 0, otherwise the relative position of the first object relative to the second object at time t2 is pt2 ;

S48, adjusting the position of the first object and/or the second object, and repeating Steps S41-S46.

FIG.1

FIG.2

FIG.3

arranging a magnetic field generating device coaxially with a first object, setting a reference area to be aligned on a second object, and determining a center point of the reference area; and arranging a plurality of magnetic sensors on the second object where a distance from each magnetic sensor to the center point is equal — S11

generating an alternating magnetic field by the magnetic field generating device, and independently detecting magnetic induction intensity in real time by the magnetic sensors — S12

comparing the magnetic induction intensity detected in real time by the magnetic sensors — S13

more than half of the magnetic sensors detect the same magnetic induction intensity in real time or the magnetic induction intensity difference is less than a preset threshold or proportional threshold? — S14

N

S15

Y

adjusting position of first object and/or second object

determining that current first object is aligned with reference area on second object，and ending positioning

FIG.4

arranging a magnetic field generating device coaxially with a first object, setting a
reference area to be aligned on a second object, and determining a center point of the
reference area; and arranging a plurality of magnetic sensors on the second object, and
acquiring a position relationship between each magnetic sensor and the center point

S21

generating an alternating magnetic field by magnetic field generating device, and
independently detecting magnetic induction intensity in real time by magnetic sensors

S22

calculating a position coordinate of each magnetic sensor relative to magnetic
field generating device according to detection data of magnetic induction intensity

S23

position coordinate of the one or less than
half of the magnetic sensors deviate from a plane determined by
position coordinates of remaining magnetic sensors?

S24

N

Y

obtaining position coordinates of center point
relative to first object according to the position
coordinates of all or any part of magnetic sensors
relative to magnetic field generating device and
position relationship between all or any part of
magnetic sensors and center point

excluding one or less than half of the magnetic sensors
as an interfered magnetic sensor

obtaining position coordinate of center point relative to first object only
according to the position coordinates of the remaining magnetic sensors
relative to magnetic field generating device and position relationships
between remaining magnetic sensors and center point

in a three-dimensional coordinate of x/y/z
coordinate axes of the center point relative to the first object, the
coordinates of two coordinate axes parallel to a plane where the first
object is located are 0 or within a preset range close to 0?

S25

Y

N

adjusting position of first object
and/or second object

S26

determining that the first object at a current
position is aligned with the reference area on the
second object, and ending positioning

FIG.5

## INTERNATIONAL SEARCH REPORT

| | International application No. |
|---|---|
| | **PCT/CN2020/133372** |

**A.    CLASSIFICATION OF SUBJECT MATTER**

A61B 6/08(2006.01)i;  G01S 1/00(2006.01)i

According to International Patent Classification (IPC) or to both national classification and IPC

**B.    FIELDS SEARCHED**

Minimum documentation searched (classification system followed by classification symbols)

A61B 6/-; G01S 1/-

Documentation searched other than minimum documentation to the extent that such documents are included in the fields searched

Electronic data base consulted during the international search (name of data base and, where practicable, search terms used)

CNPAT, CNKI, WPI, EPODOC: 精确定位, 阈值, 交变磁场, 磁定位, 正对, 对齐, 江苏康众数字医疗科技股份有限公司, X光源, 球管, 磁场定位, 对准, 射线源, 磁场, 电磁跟踪, sensor?, generator, SID, source, X-ray, magnetic

**C.    DOCUMENTS CONSIDERED TO BE RELEVANT**

| Category* | Citation of document, with indication, where appropriate, of the relevant passages | Relevant to claim No. |
|---|---|---|
| X | CN 103415252 A (CARESTREAM HEALTH INC.) 27 November 2013 (2013-11-27) description paragraphs [0001], [0064], [0065], [0087]-[0089], [0092], [0093], [0095], [0105],[0106], figures 1-3, 9, 11-20 | 10 |
| Y | CN 103415252 A (CARESTREAM HEALTH INC.) 27 November 2013 (2013-11-27) description paragraphs [0001], [0064], [0065],[0087]-[0089], [0092], [0093],[0095], [0105],[0106], figures 1-3, 9, 11-20 | 1-9, 11-14 |
| Y | CN 110293860 A (BEIJING INVISPOWER CO., LTD.) 01 October 2019 (2019-10-01) description paragraphs [0101], [0110], [0116], [0117], [0119], figures 1-4 | 1-9, 11-14 |
| A | CN 109451773 A (QUALCOMM INC.) 08 March 2019 (2019-03-08) entire document | 1-14 |
| A | CN 102262167 A (ZHANG, Feng) 30 November 2011 (2011-11-30) entire document | 1-14 |
| A | CN 110602988 A (AGFA HEALTHCARE) 20 December 2019 (2019-12-20) entire document | 1-14 |
| A | DE 102010008551 A1 (SIEMENS AG.) 25 August 2011 (2011-08-25) entire document | 1-14 |

☐ Further documents are listed in the continuation of Box C.  ☑ See patent family annex.

| \* | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 February 2021** | **16 March 2021** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/133372**

| Patent document cited in search report | | | Publication date (day/month/year) | Patent family member(s) | | | Publication date (day/month/year) |
|---|---|---|---|---|---|---|---|
| CN | 103415252 | A | 27 November 2013 | US | 9179886 | B2 | 10 November 2015 |
| | | | | US | 2012230473 | A1 | 13 September 2012 |
| | | | | WO | 2012121890 | A2 | 13 September 2012 |
| | | | | JP | 2014507247 | A | 27 March 2014 |
| | | | | US | 2015049863 | A1 | 19 February 2015 |
| | | | | EP | 2683299 | A2 | 15 January 2014 |
| | | | | CN | 103415252 | B | 13 April 2016 |
| | | | | US | 8821015 | B2 | 02 September 2014 |
| CN | 110293860 | A | 01 October 2019 | CN | 210852086 | U | 26 June 2020 |
| CN | 109451773 | A | 08 March 2019 | WO | 2017218093 | A1 | 21 December 2017 |
| | | | | US | 10029577 | B2 | 24 July 2018 |
| | | | | US | 2017361726 | A1 | 21 December 2017 |
| CN | 102262167 | A | 30 November 2011 | | None | | |
| CN | 110602988 | A | 20 December 2019 | US | 2020107798 | A1 | 09 April 2020 |
| | | | | EP | 3606430 | A1 | 12 February 2020 |
| | | | | US | 2020107799 | A1 | 09 April 2020 |
| | | | | WO | 2018184923 | A1 | 11 October 2018 |
| | | | | EP | 3606429 | A1 | 12 February 2020 |
| | | | | CN | 110536639 | A | 03 December 2019 |
| | | | | WO | 2018184705 | A1 | 11 October 2018 |
| DE | 102010008551 | A1 | 25 August 2011 | DE | 102010008551 | B4 | 06 February 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2020105471548